# EUROPEAN PATENT APPLICATION

(11) **EP 3 991 633 A1**
(43) Date of publication of application: **04.05.2022**
(21) Application number: 20205392.2
(22) Date of filing: 03.11.2020
(51) Int. Cl.: A61B 3/00, A61B 3/13, A61B 3/14

(54) **MICROSCOPE SYSTEM FOR USE IN EYE SURGERY AND CORRESPONDING SYSTEM, METHODS AND COMPUTER PROGRAMS**

(71) Applicant: Leica Instruments (Singapore) Pte. Ltd., 608924 Singapore (SG)
(72) Inventor: SCHUTZ, Marco, 338986 Singapore (SG); YANG, Gao, 650206 Singapore (SG)
(74) Representative: 2SPL Patentanwälte PartG mbB

(57) **Abstract**

Examples relate to a use of infrared illumination in ophthalmic microscopy, and in particular microscope system for use in eye surgery and to a corresponding system, to corresponding methods and to corresponding computer programs. The microscope system comprises a microscope comprising at least one optical imaging sensor. The microscope system comprises an illumination system for illuminating the eye. The illumination system is configured to emit light in a near infrared wavelength band and in a visible light wavelength band. The microscope system comprises one or more processors configured to obtain image data of the least one optical imaging sensor of the microscope. The image data represents infrared light and visible light sensed by the at least one optical imaging sensor. The one or more processors are configured to process the image data to determine a first component representing visible light and a second component representing infrared light. The one or more processors are configured to generate processed image data based on the first component and based on the second component of the image data. The one or more processors are configured to generate a display signal for a display device based on the processed image data.

## Description

### Technical field

Examples relate to a use of infrared illumination in ophthalmic microscopy, and in particular to a microscope system for use in eye surgery and to a corresponding system, to corresponding methods and to corresponding computer programs.

### Background

Ophthalmic microscopes provide surgeons with a magnified view of the patient's eye during operation. The eye needs to be illuminated for good visibility. In general, visible light is used to illuminate the eye through either a coaxial light path or an endoilluminator. For posterior surgeries, high doses of light can damage the retina and cause irreversible vision loss. At retina-safe light levels, the visibility through the optical binoculars may be reduced, while videos from microscope cameras may be noisy. Light sources with reduced blue light component result in poor color rendition and low visibility of retinal dyes.

### Summary

There may be a desire to provide an improved concept for a microscope system for use in eye surgery.

This desire is addressed by the subject-matter of the independent claims.

Various embodiments of the present disclosure are based on the finding, that near infrared light is less toxic to retina than visible light and can be safely applied at higher doses. At the same time, infrared light can be recorded using common components, as electronic image sensors are sensitive to near infrared light in addition to visible light, unless used in combination with an infrared filter. By recording the infrared light and the visible light, an overall higher level of illumination can be provided and recorded, which leads to clearer imagery.

To utilize both the visible light and the infrared light, image processing is applied to the resulting image data, which comprises a first component that represents the visible light and a second component that represents the infrared light, and processed image data is generated by generating a combined image based on the two components. The processed image data can then be output via a display of the microscope system, e.g. via a heads-up display. Therefore, near infrared illumination can be used to augment visualization in a digital heads-up surgery setup.

Various embodiments of the present disclosure relate to a microscope system for use in eye surgery (ophthalmic surgery). The microscope system comprises a microscope comprising at least one optical imaging sensor. The microscope system comprises an illumination system for illuminating the eye. The illumination system is configured to emit light in a near infrared wavelength band and in a visible light wavelength band. The microscope system comprises one or more processors configured to obtain image data of the least one optical imaging sensor of the microscope. The image data represents infrared light and visible light sensed by the at least one optical imaging sensor. The one or more processors are configured to process the image data to determine a first component representing visible light and a second component representing infrared light. The one or more processors are configured to generate processed image data based on the first component and based on the second component of the image data. The one or more processors are configured to generate a display signal for a display device based on the processed image data. By using both visible light and infrared light, and performing image processing on the two components, a color acuity of the processed image data can be maintained, while reducing noise of the resulting image.

There are various approaches to providing the illumination towards the eye. For example, the illumination system may be configured to provide the illumination through a co-axial light path of the microscope. Alternatively, the illumination system may be configured to provide the illumination via an endo-illuminator. For example, a light source of the endo-illuminator may be integrated in a stand of the microscope system or within the microscope. By using the co-axial light path of the microscope, the illumination can be applied without the need for an additional instrument to be arranged in proximity of the eye. However, if an endo-illuminator is used, a more precise illumination of the eye is enabled.

In some examples, the image data comprises the first component representing visible light and the second component representing infrared light separately from each other. This can be achieved by using separate sensors for infrared light and visible light. In this case, no additional separation of the two components by the one or more processors may be required, which may reduce a delay caused by the image processing.

Alternatively, the one or more processors may be configured to separate the first and the second component within the image data. For example, if infrared light is sensed only by a subset of pixels, e.g. due to the application of a per-pixel infrared or polarization filter, the two components can be separated by the one or more processors.

In general, the image data may represent infrared light and visible light reflected by the eye and subsequently sensed by the at least one optical imaging sensor. In other words, the proposed concept refers to reflectance imaging, and might not refer to fluorescence imaging.

In some examples, the one or more processors are configured to spatially low-pass filter the first component and to spatially high-pass filter the second component, and to generate the processed image data by combining the spatially low-pass filtered first component and the spatially high-pass filtered second component. By low-pass filtering the first component, an averaging effect is applied on the visible light component of the image data, which leads to reduced noise in continuous areas of the resulting processed image data. By high-pass filtering the second component, edges within the image data are emphasized, which leads to clear edges in the resulting processed image data.

Alternatively or additionally, the infrared component may be used to adapt a filter that is used to filter the visible light components. For example, the one or more processors may be configured to determine a weighting matrix based on the second component, to use the weighting matrix as spatially varying filter kernels for filtering the first component, and to generate the processed image data based on the filtered first component. This may also reduce noise, while retaining the color acuity, within the processed image data.

As mentioned above, in some examples, a separate image sensor may be used to capture the second component. In other words, the microscope may comprise at least one optical imaging sensor for sensing visible light and a separate optical imaging sensor for sensing infrared light. By using separate sensors, the image data may be obtained such, that the first component representing visible light and the second component representing infrared light are included separately from each other, so no separation is to be performed by the one or more processors.

In this case, the visible light may be recorded using a single sensor or using a three-sensor system. In other words, the microscope may comprise a single optical imaging sensor for sensing visible light and the separate optical imaging sensor for sensing infrared light. Alternatively, the microscope may comprise three optical imaging sensors for sensing visible light and the separate optical imaging sensor for sensing infrared light. A microscope system with a single sensor for capturing visible light (or rather a single sensor per light path, if stereoscopic imaging is used) can be built more compactly, while an effective resolution of a microscope system with three optical sensors (per light path) for sensing visible light may be higher, as the image data may be used without applying demosaicing on the raw image data.

Alternatively, the microscope may comprise at least one optical imaging sensor for simultaneously sensing visible light and infrared light. In this case, various filters may be included in the microscope system, so that the infrared light is sensed (only) by a subset of pixels of the respective at least one optical imaging sensor.

In some examples, the at least one optical imaging sensor comprises a polarization filter configured to admit light having a first polarization to a first subset of pixels of the at least one optical imaging sensor and to admit light having a second polarization to a second subset of pixels of the at least one optical imaging sensor, the first polarization being orthogonal to the second polarization. The illumination system may be configured to emit the light in the visible light wavelength band having the first polarization, and to emit the light in the near infrared wavelength band having the second polarization. In other words, due to the different polarization of the visible light and the infrared light, the first subset of pixels might only capture visible light, and the second subset of pixels might only capture infrared light.

Alternatively, the at least one optical imaging sensor may comprise an infrared light filter configured to admit infrared light to a subset of pixels of the at least one optical imaging sensor. In other words, per-pixel infrared filters may be applied, e.g. to block infrared light from the first subset of pixels of the at least one optical imaging sensor and to admit infrared light to the second subset of pixels of the at least one optical imaging sensors.

In some systems, three optical imaging sensors may be used. The infrared light filter may be configured to admit infrared light to a subset of pixels of each of the three optical imaging sensors, such that infrared light is admitted to a different subset of pixels of each of the three optical imaging sensors. This may increase the spatial resolution of the second component representing infrared light. The same concept can be applied to the polarization filters. The polarization filter may be configured to admit light having the second polarization to a subset of pixels of each of the three optical imaging sensors, such that light having the second polarization (i.e. infrared light) is admitted to a different subset of pixels of each of the three optical imaging sensors.

In various examples, the microscope system comprises a heads-up display configured to display the processed image data. On large heads-up displays, an effective removal of image noise and clear display of the microscope image is particularly beneficial.

In some examples, the light in the near infrared wavelength band is light having a wavelength between 730 nm and 790 nm or above 900 nm. In these wavelength ranges, fluorescence emissions or excitation wavelengths of common fluorophores may be excluded, so fluorescence imaging is possible in addition.

In various examples, the one or more processors correspond to an application-specific processor for processing image data of a microscope system. By using an application-specific processor, an efficiency of the image processing may be improved.

Various embodiments of the present disclosure relate to a corresponding method for a microscope system for use in eye surgery. The method comprises emitting light in a near infrared wavelength band and in a visible light wavelength band. The method comprises obtaining image data of at least one optical imaging sensor of the microscope, the image data representing infrared light and visible light sensed by the at least one optical imaging sensor. The method comprises processing the image data to determine a first component representing visible light and a second component representing infrared light. The method comprises generating processed image data based on the first component and based on the second component of the image data. The method comprises generating a display signal based on the processed image data.

Various aspects of the present disclosure provide a system for a microscope system for use in eye surgery. The system comprises (the) one or more processors and one or more storage devices. The system is configured to obtain image data of at least one optical imaging sensor of a microscope of the microscope system, the image data representing infrared light and visible light sensed by the at least one optical imaging sensor. The system is configured to process the image data to determine a first component representing visible light and a second component representing infrared light. The system is configured to generate processed image data based on the first component and based on the second component of the image data. The system is configured to generate a display signal for a display device based on the processed image data.

Various aspects of the present disclosure provide a corresponding method for a system for a microscope system. The method comprises obtaining image data of at least one optical imaging sensor of the microscope. The image data represents infrared light and visible light sensed by the at least one optical imaging sensor of a microscope of the microscope system. The method comprises processing the image data to determine a first component representing visible light and a second component representing infrared light. The method comprises generating processed image data based on the first component and based on the second component of the image data. The method comprises generating a display signal based on the processed image data.

Various aspects of the present disclosure provide a corresponding computer program with a program code for performing one of the above methods, when the computer program is executed on a processor.

### Short description of the Figures

Some examples of apparatuses and/or methods will be described in the following by way of example only, and with reference to the accompanying figures, in which
- Fig. 1a: shows a schematic diagram of an example of a microscope system for use in eye surgery;
- Fig. 1b: shows a block diagram of an example of a system for a microscope system;
- Fig. 2: shows a schematic diagram of an example of a method for a microscope system and/or of a method for a system of a microscope system.
- Fig. 3a: shows a schematic diagram of a sensor arrangement comprising three sensors for sensing visible light and a separate sensor for sensing infrared light;
- Fig. 3b: shows a schematic diagram of a sensor arrangement comprising a single sensor for sensing visible light and a separate sensor for sensing infrared light;
- Fig. 3c: shows a schematic diagram of a sensor arrangement comprising three sensors for simultaneously sensing visible light and infrared light;
- Fig. 3d: shows a schematic diagram of a sensor arrangement comprising a single sensor for simultaneously sensing visible light and infrared light;
- Fig. 3e: shows a schematic diagram of filters being applied to three sensors for simultaneously sensing visible light and infrared light; and
- Fig .4: shows a schematic diagram of a system comprising a microscope and a computer system.

### Detailed Description

Various examples will now be described more fully with reference to the accompanying drawings in which some examples are illustrated. In the figures, the thicknesses of lines, layers and/or regions may be exaggerated for clarity.

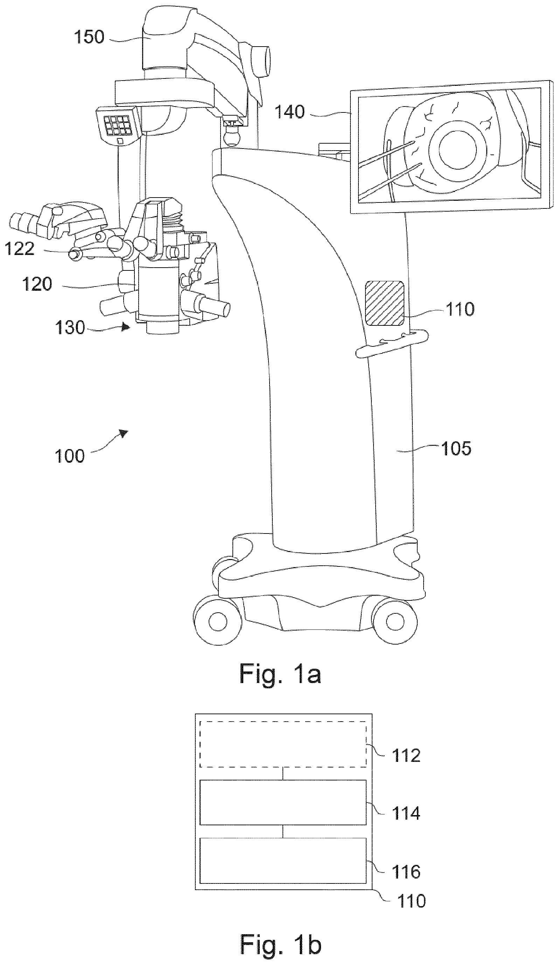

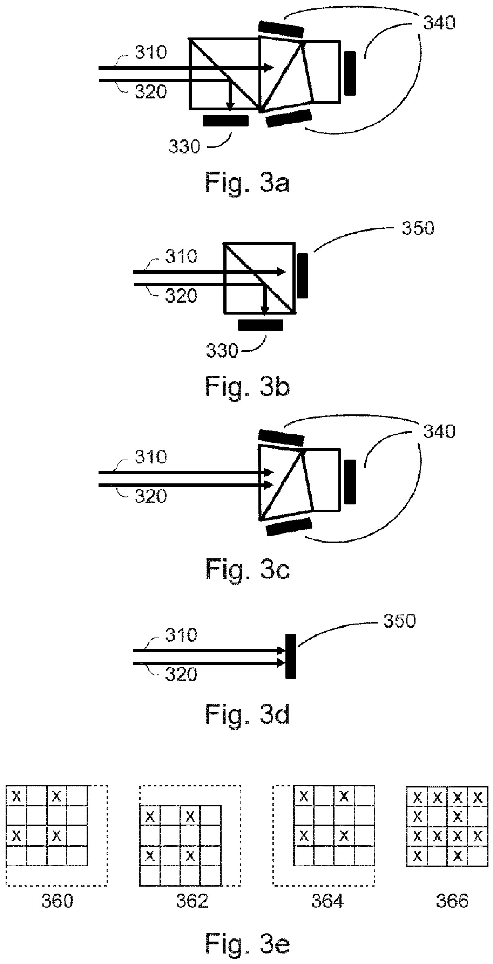

Fig. 1a shows a schematic diagram of an example of a microscope system 100 for use in eye surgery (ophthalmic surgery). The microscope system comprises a microscope 120, the microscope 120 comprising at least one optical imaging sensor 330; 340; 350 (shown in Figs. 3a to 3e). The microscope system comprises an illumination system 130 for illuminating the eye. The illumination system is configured to emit light in a near infrared wavelength band and in a visible light wavelength band. The microscope system 110 may comprise one or more additional, optional features, such as a base unit / stand 105 (that comprises a system 110 with the one or more processors 114), and an arm 150 that the microscope 120 is attached to. For example, the microscope system shown in Fig. 1a comprises ocular eyepieces 122 and an (external) auxiliary display 140, which may both be used as display device of the microscope system. Alternatively, a display device of the operating room may be used. In other words, the display device may be a display device of the microscope system, or a display device that is external to the microscope system, e.g. a separate display on a cart system or even an already installed display inside the operating room/hospital. For example, the auxiliary display 140 may be a heads-up display, i.e. a display being used by a surgeon instead of the oculars. Accordingly, the microscope system may comprise a heads-up display 140 configured to display the processed image data. The microscope system shown in Fig. 1a is a surgical microscope system, which may be used at a surgical site by a surgeon. In particular, the microscope system shown in Fig. 1a is a surgical microscope system for use in ophthalmology (eye surgery).

The microscope system further comprises one or more processors 114. For example, the one or more processors may be part of a system 110 for a microscope system. The system 110 comprises the one or more processors 114 and one or more storage devices 116. The system 110 may further comprise an interface 112 for connecting the system, and in particular the one or more processors, with other components of the microscope system 100, such as the at least one optical imaging sensor and/or the illumination system. The one or more processors 114 are coupled to the optional interface 112 and the one or more storage devices 116. For example, the microscope system may comprise the system 110. In general, the functionality of the system 110 is provided by the one or more processors 114, e.g. in conjunction with the optional interface 112 and/or the one or more storage devices 116. For example, the one or more processors may correspond to an application-specific processor for processing image data of a microscope system, e.g. to an image processing processor or digital signal processor for image processing.

The one or more processors (and thus the system 110) are configured to obtain image data of the least one optical imaging sensor of the microscope (e.g. via the interface 112). The image data represents infrared light and visible light sensed by the at least one optical imaging sensor. The one or more processors (and thus the system 110) are configured to process the image data to determine a first component representing visible light and a second component representing infrared light. The one or more processors (and thus the system 110) are configured to generate processed image data based on the first component and based on the second component of the image data. The one or more processors (and thus the system 110) are configured to generate a display signal for a display device 122; 140 based on the processed image data. Fig. 1b shows a block diagram of an example of such a system 110 for a microscope system 100.

Embodiments of the present disclosure relate to a microscope system, and to a system, a method and a computer program that are suitable for a microscope system, such as the microscope system 100 introduced in connection with Fig. 1b. As has been introduced above, a distinction is made between the microscope 120 and the microscope system 100, with the microscope system comprising the microscope 120 and various components that are used in conjunction with the microscope 120, e.g. the illumination system 130 and the auxiliary display 140 etc. In a microscope system, the actual microscope is often also referred to as the "optical carrier", as it comprises the optical components of the microscope system. In general, a microscope is an optical instrument that is suitable for examining objects that are too small to be examined by the human eye (alone). For example, a microscope may provide an optical magnification of an object. In modern microscopes, the optical magnification is often provided for a camera or an imaging sensor. The microscope 120 may further comprise one or more optical magnification components that are used to magnify a view on the sample.

There are a variety of different types of microscopes. If the microscope system is used in the medical or biological fields, the object being viewed through the microscope may be a sample of organic tissue, e.g. arranged within a petri dish or present in a part of a body of a patient. In particular, the microscope system shown in Fig. 1a is a microscope system fur use during eye surgery, i.e. a surgical microscope system for ophthalmic surgery.

The proposed concept is based on a combination of the illumination system that is used to simultaneously provide visible light and infrared illumination, the at least one optical imaging sensor, and the one or more processors that process the image data that is provided by the at least one image sensor to provide a visible light image with an image quality that is enhanced based on the additional information gained from the infrared illumination.

Accordingly, the microscope system comprises the illumination system 130 that is suitable for illuminating the eye, by emitting light in the near infrared wavelength band (e.g. between 750 nm and 1.4 µm) and in a visible light wavelength band (between 380 nm and 740 nm). For example, the illumination system may comprise a first light source for providing the infrared illumination and a second light source for providing the near infrared illumination. Alternatively, the illumination system may comprise a single broad-spectrum light source (or a single type of light sources, if multiple Light-Emitting Diodes (LEDs) are used) that is configured to emit light across the visible light and visible light spectrum. In various embodiments, the light emitted by the illumination system may cover major portions of both spectra, e.g. light across at least 250 nm of spectrum within the visible light wavelength band, and/or light across at least 100 nm of spectrum within the near infrared wavelength band. In general, the light in the near infrared wavelength band and the light in the visible light wavelength band may be emitted substantially simultaneously. For example, in a given time interval (e.g. in a given second), both the light in the near infrared wavelength band and the light in the visible light wavelength band may be emitted by the illumination system. For example, the light in the near infrared wavelength band and the light in the visible light wavelength band may be mixed together, or emitted separately, e.g. using different polarizations, or in a time-separated manner (e.g. using a filter wheel or by modulating the light sources).

In some cases, the light that is emitted towards the eye may be further restricted regarding the spectrum being used. For example, some microscope systems support fluorescence imaging, in which light in a fluorescence excitation wavelength band is emitted towards a sample (i.e. the eye), a fluorophore is excited and light in a fluorescence emission wavelength band is emitted by the sample (i.e. the fluorophore within the sample). In this case, the fluorescence emission and/or the fluorescence excitation wavelength band(s) may be optionally or permanently blocked within the light emitted by the illumination system. For example, the illumination system may comprise one or more bandpass filters for blocking one or more portions of the spectrum.

One of the fluorophores being commonly used during surgery is Indocyanine Green (ICG), which has an absorption maximum (in the fluorescence excitation wavelength band) at ca. 800 nm, and a fluorescence emission maximum at ca. 810 nm in water, and 830 nm in blood, with fluorescence emissions going up to approximately 950 nm. To enable fluorescence imaging via the ICG fluorophore, the light emitted in the near infrared wavelength band may be limited. For example, the light in the near infrared wavelength band may be light having a wavelength between 730 nm and 790 nm, or above 900 nm.

There are various concepts for providing the light that is emitted towards the eye from the light sources or light source of the illumination system to the eye. For example, the illumination system may be configured to provide the illumination through a co-axial light path of the microscope, e.g. at least partially using the same light path (in the opposite direction) as light captured by the at least one optical imaging sensor. For example, the light may be generated within the microscope 120, or be coupled into the microscope 120 using a light conductor (with the light being generated within the base unit / stand 105 of the microscope system). Alternatively, the illumination system may be configured to provide the illumination via an endo-illuminator. For example, the endo-illuminator may be part of the illumination system, or it may be coupled to the illumination system. An endo-illuminator is a light-conduction tool (for use in eye surgery, for example), which can be used to conduct light from a light source to an illumination target. In eye surgery, the endo-illuminator is attached to the eye, e.g. piercing through the sclera and choroid, to provide illumination during eye surgery. For example, a light source of the endo-illuminator may be integrated in a stand of the microscope system or within the microscope.

The microscope 120 comprises the at least one optical imaging sensor 330; 340; 350 (shown in Figs. 3a to 3c). For example, the at least one optical imaging sensor of the microscope may comprise or be an APS (Active Pixel Sensor) - or a CCD (Charge-Coupled-Device)-based imaging sensor. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the optical imaging sensor by a control circuitry of the optical imaging sensor to perform the imaging. The image data may be obtained by receiving the image data from the at least one optical imaging sensor (e.g. via the interface 112 and/or the system 130), by reading the image data out from a memory of the at least one optical imaging sensor (e.g. via the interface 112), or by reading the image data from a storage device 116 of the system 110, e.g. after the image data has been written to the storage device 116 by the at least one optical imaging sensor or by another system or processor.

In the context of the present disclosure, for simplicity, the microscope is discussed with singular terms for "light path/optical path" "optical imaging sensor" "eyepiece" etc. It will be appreciated that microscopes are often implemented as co-axial microscopes, with a co-axial light path, an eyepiece with two oculars, and two optical imaging sensors (or two groups of optical imaging sensors) for capturing the light guided on the co-axial light path. The singular terms used in the context of the present disclosure thus also refer to the respective components of a co-axial implementation of the microscope.

The one or more processors are configured to obtain the image data of the least one optical imaging sensor of the microscope, with the image data representing infrared light and visible light sensed by the at least one optical imaging sensor. As has been mentioned above, some microscope system support "fluorescence imaging", with some of the fluorophores having emission wavelength bands that reach into the infrared spectrum. However, these fluorescence emissions in the infrared band might not be included in the generation of the processed image data. Instead, the image data may be the result of reflectance imaging (and not of fluorescence imaging). In other words, the image data may represent infrared light and visible light reflected by the eye and subsequently sensed by the at least one optical imaging sensor. Also, the processed image data may be generated particularly based on the component of the image data that relates to infrared light that is emitted by the illumination system and reflected by the eye.

There are various sensor configurations suitable for use with the proposed concept. The following configurations are discussed with reference to Figs. 3a to 3e. In some sensor configurations, a separate optical imaging sensor is used to sense the infrared light. In other words, the microscope may comprise at least one optical imaging sensor 340; 350 for sensing visible light and a separate optical imaging sensor 330 for sensing infrared light. For example, the at least one optical imaging sensor 340; 350 for sensing visible light may be coupled with an infrared filter for blocking infrared light. Again, two main configurations are possible - with a single sensor, e.g. a Red-Green-Blue-sensor with a Bayer filter, for sensing the visible light, or with multiple sensors (e.g. three sensors) for sensing light in mutually different wavelength bands. In other words, as shown in Fig. 3b, the microscope may comprise a single optical imaging sensor 350 for sensing visible light and the separate optical imaging sensor 330 for sensing infrared light. Alternatively, as shown in Fig. 3a, the microscope may comprise three optical imaging sensors 340 for sensing visible light and the separate optical imaging sensor 330 for sensing infrared light.

Alternatively, the same sensors may be used for sensing infrared light and visible light. In other words, the microscope may comprise at least one optical imaging sensor 340; 350 for simultaneously sensing visible light and infrared light. In other words, the at least one optical imaging sensor may be configured to sense both visible light and infrared light during a given time-interval (e.g. Is, if the illumination system alternates between emitting visible light and infrared light over time). In other words, each of the at least one optical imaging sensor may be configured to sense both visible light and infrared light. Again, a one-sensor or three-sensor approach may be chosen. In other words, the microscope may comprise a single optical imaging sensor configured to simultaneously sense visible light and infrared light, as shown in Fig. 3d. Alternatively, the microscope may comprise three optical imaging sensors, with each optical imaging sensor being configured to simultaneously sense visible light and infrared light. To separate the visible light and infrared light incident to the sensor, each pixel of the at least one optical imaging sensor may be configured to sense either visible light or infrared light, e.g. in combination with appropriate filters that affect only a subset of pixels each. Alternatively, at any given time, the at least one optical image sensor may sense either visible light or infrared light, based on the time-alternating emission of visible light and infrared light by the illumination system. Consequently, the image data may represent, in any given frame of the image data, either visible light or infrared light.

For example, the visible light and the infrared light may be emitted with different polarizations, e.g. polarizations that are orthogonal to each other. In other words, the illumination system may be configured to emit the light in the visible light wavelength band with a first polarization, and to emit the light in the near infrared wavelength band with the second polarization, e.g. via polarization filters that are coupled with the respective light sources of the illumination system. For example, the first polarization may be orthogonal to the second polarization. The pixels of the at least one optical imaging sensor may be coupled with polarization filters (e.g. on a pixel-by-pixel basis), configured to admit either visible light (i.e. a light having the polarization being used for the visible light) or infrared light (i.e. light having the polarization being used for the infrared light). In other words, the at least one optical imaging sensor may comprise a polarization filter configured to admit light having the first polarization (and thus visible light) to a first subset of pixels of the at least one optical imaging sensor and to admit light having the second polarization (and thus infrared light) to a second subset of pixels of the at least one optical imaging sensor. Within the image data, the first subset of pixels may represent visible light, and the second subset of pixels may represent the infrared light.

Alternatively, an infrared filter (or infrared filters) may be used to admit the infrared light to some of the pixels, and to block it from others. In other words, the at least one optical imaging sensor may comprise an infrared light filter configured to admit infrared light to a subset of pixels (e.g. the second subset of pixels) of the at least one optical imaging sensor. Accordingly, the infrared filter may be configured to block infrared light from the remaining pixels (e.g. the first subset of pixels). Again, within the image data, the first subset of pixels may represent visible light, and the second subset of pixels may represent the infrared light.

If multiple image sensors are used to simultaneously sense visible light and infrared light, the distribution of the pixels of the first subset and the second subset of pixels may be adapted to improve the resolution of the second component of the image data (representing the infrared light). For example, the microscope may comprise three optical imaging sensors (as shown in Fig. 3c). As illustrated in Fig. 3e, the infrared light filter may be configured to admit infrared light to a subset of pixels of each of the three optical imaging sensors, such that infrared light is admitted to a different subset of pixels of each of the three optical imaging sensors. In Fig. 3e, the pixels that infrared light is admitted to are marked with an "X". By placing the pattern of X's 360; 362; 364 differently in each sensor, a combined pattern 366 is generated. If the image data of the sensors is combined, for three of four pixels of the image data, the image data comprises information on the visible light incident to the corresponding pixels of the sensors, and infrared light incident to the corresponding pixels of the sensors.

As has been pointed out above, the optical image sensor(s) have been described with reference to a single optical path. In a co-axial microscope, each of the sensors may be included twice, once for each light path of the co-axial light path.

The one or more processors are configured to process the image data to determine the first component representing the visible light and the second component representing infrared light. For example, in some configurations, the image data may comprise the first component representing visible light and the second component representing infrared light separately from each other. This may, for example, be the case if a separate optical imaging sensor is being used to capture the second component of the image data.

If the same optical imaging sensor or optical imaging sensors is/are being used to sense the first and second component (albeit either/or on a pixel-by-pixel or frame-by-frame basis), the first and second component may be extracted by the one or more processors. In other words, the one or more processors are configured to separate the first and the second component within the image data. For example, the one or more processors may be configured to determine the first component based on the first subset of pixels, and to determine the second component based on the second subset of pixels. In this case, the optical imaging sensor(s) might not perform demosaicing, or the demosaicing procedure of the optical imaging sensor(s) may be adapted based on the first and second subset of pixels. For example, if the demosaicing is not performed by the optical imaging sensor(s), the one or more optical imaging sensors may be configured to perform demosaicing separately for the first and for the second subset of pixels. Alternatively, the one or more processors may be configured to determine the first component and the second component depending on the light being emitted in a given frame by the illumination system.

The one or more processors are configured to generate the processed image data based on the first component and based on the second component of the image data. In other words, the one or more processors may be configured to generate a combined image (i.e. the processed image data) based on the first component and based on the second component. In this context, the processed image data might not be generated by generating a color overlay (representing the infrared light) that is overlaid over the first component. On the contrary, the second component may be used to de-noise the first component, or different portions of the first and second component may be combined to generate the processed image data.

For example, the second component may be used to de-noise the first component. In this context, the second component may be used as "guide image" for the de-noising of the first component. In particular, the one or more processors may be configured to determine a weighting matrix based on the second component. To determine the weighting matrix, each pixel in the second component may be compared with its adjacent pixels, with more similar neighbors resulting in larger weights. The one or more processors may be configured to use the weighting matrix as spatially varying filter kernels for filtering the first component. For example, filter kernels that are based on pixels receiving larger weights may cause the one or more processors to apply stronger denoising on the corresponding pixel or pixels of the first component than filter kernels that are based on pixels receiving lower weights. In other words, the weighting matrix may determine the severity of the de-noising being applied to the corresponding pixels or portions of the first component. The one or more processors may be configured to generate the processed image data based on the filtered first component.

Alternatively or additionally, different portions of the first and second component may be combined to generate the processed image data. In this context, the second component may be used as "detail image" for the processed image data. For example, large continuous areas may be taken from the first component (representing the correct color of the large continuous areas), with de-noising being applied on the large areas taken from the first component, and edges may be taken from the second component. To achieve this, the first component may be spatially low-pass filtered, thus providing de-noised continuous areas for the processed image data, and the second component may be spatially high-pass filtered, thus providing the edges for the processed image data. In other words, the one or more processors may be configured to spatially low-pass filter the first component and to spatially high-pass filter the second component, and to generate the processed image data by combining the spatially low-pass filtered first component and the spatially high-pass filtered second component. In this context, the term "spatial" low-pass or high-pass filter indicates, that the filter is being applied on the difference between adjacent pixels.

The one or more processors are configured to generate the display signal for the display device 122; 140 of the microscope system based on the processed image data. In other words, the display signal may comprise, or may be based on, the processed image data. The one or more processors may be configured to provide the display signal to the display device, e.g. to the ocular displays 122 or to a heads-up display 140 of the microscope system, or to the separate display. For example, the display signal may be provided to the display device via the interface 112.

The interface 112 may correspond to one or more inputs and/or outputs for receiving and/or transmitting information, which may be in digital (bit) values according to a specified code, within a module, between modules or between modules of different entities. For example, the interface 112 may comprise interface circuitry configured to receive and/or transmit information. In examples the one or more processors 114 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. In at least some examples, the one or more storage devices 116 may comprise at least one element of the group of a computer readable storage medium, such as an magnetic or optical storage medium, e.g. a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

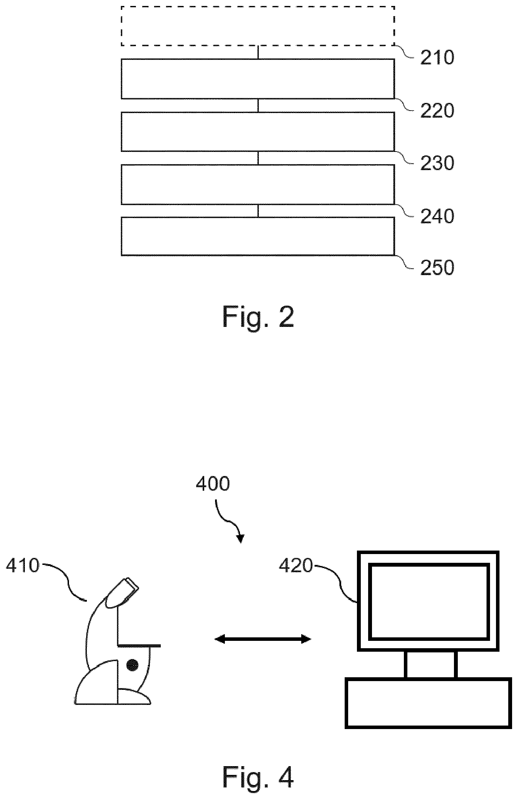

More details and aspects of the microscope system and of the system, method and computer program for the microscope system are mentioned in connection with the proposed concept or one or more examples described above or below (e.g. Fig. 2 to 4). The microscope system and of the system, method and computer program for the microscope system may comprise one or more additional optional features corresponding to one or more aspects of the proposed concept or one or more examples described above or below.

Fig. 2 shows a schematic diagram of an example of a method for a microscope system and/or of a method for a system of a microscope system. In Fig. 2, both the method for the microscope system and the method for the system for the microscope system are shown, as the system for the microscope system may be used to perform various features of the method for the microscope system. For examples, the system may correspond to the system 110 and the microscope system may correspond to the microscope system 100 introduced in connection with Figs. 1a and 1b.

The method for the microscope system comprises emitting 210 (e.g. by the illumination system 130) light in a near infrared wavelength band and in a visible light wavelength band. The method comprises obtaining 220 (e.g. by the one or more processors 114 and via the interface 112) image data of at least one optical imaging sensor of the microscope, the image data representing infrared light and visible light sensed by the at least one optical imaging sensor. The method comprises processing 230 (e.g. by the one or more processors) the image data to determine a first component representing visible light and a second component representing infrared light. The method comprises generating 240 (e.g. by the one or more processors) processed image data based on the first component and based on the second component of the image data. The method comprises generating 250 (e.g. by the one or more processors) a display signal based on the processed image data. For example, the method may comprise providing (e.g. by the one or more processors via the interface) the display signal to the display device.

The method for the system for the microscope system comprises obtaining 220 the image data of the at least one optical imaging sensor of the microscope, the image data representing infrared light and visible light sensed by the at least one optical imaging sensor of a microscope of the microscope system. The method comprises processing 230 the image data to determine the first component representing visible light and the second component representing infrared light. The method comprises generating 240 the processed image data based on the first component and based on the second component of the image data. The method comprises generating 250 a display signal based on the processed image data. Optionally, the method comprises providing the display signal to the display device.

As indicated above, features described in connection with the system 110 and the microscope system 100 of Figs. 1a to 1b may be likewise applied to the methods of Fig. 2.

More details and aspects of the methods are mentioned in connection with the proposed concept or one or more examples described above or below (e.g. Fig. 1a to 1b, 3a to 4). The methods may comprise one or more additional optional features corresponding to one or more aspects of the proposed concept or one or more examples described above or below.

In the following, some of the concepts introduced in connection with Figs. 1a to 2 are introduced in more detail.

First, various methods of illumination are detailed. For example, the light source spectrum may contain both visible light and near infrared light. The infrared and visible components may be mixed or separated by time or polarization. In some examples, coaxial illumination may be used, i.e. the light may be emitted through the co-axial light path of the microscope. The light may come through the microscope's objective lens into the back of patient's eye. Alternatively, endo-illumination may be used, wherein light comes from a light source outside of the microscope or integrated with the microscope, through an optical fiber, into the back of the patient's eye. The light source spectrum may contain both visible light and near infrared light.

In the following, implementations regarding the arrangement of image sensors (i.e. the optical imaging sensors introduced in connection with Figs. 1a and 1b) are discussed.

In some examples, one additional infrared image sensor per view may be added to a conventional design. A dichroic prism may be inserted between this additional infrared image sensor (330 in Figs. 3a and 3b) and the existing sensor(s), so that infrared light is directed to the infrared sensor. Fig. 3a shows a schematic diagram of a sensor arrangement comprising three sensors 340 for sensing visible light and a separate sensor 330 for sensing infrared light. Fig. 3b shows a schematic diagram of a sensor arrangement comprising a single sensor 350 for sensing visible light and a separate sensor 330 for sensing infrared light. For example, sensor 350 may be an existing color image sensor (one-chip design). Sensors 340 may be existing color image sensors (in a three-chip design). In Figs. 3a to 3d, the visible light 310 and the infrared light 320 is shown. In Figs. 3a and 3b, the infrared light 320 is redirected by the dichroic prism towards the separate infrared sensor 330.

In some examples, the microscope may be implemented without additional image sensors (as per typical 1-chip or 3-chip color cameras). In this case, the image sensors (340/350 in Figs. 3c and 3d) might employ no infrared-cut filter or have an infrared-cut filter which allows the infrared component of the corresponding light source to pass through. Fig. 3c shows a schematic diagram of a sensor arrangement comprising three sensors for simultaneously sensing visible light and infrared light. Fig. 3d shows a schematic diagram of a sensor arrangement comprising a single sensor for simultaneously sensing visible light and infrared light. In general, without any additional filters, the sensor(s) may record infrared light and visible light simultaneously and each pixel value contains a mixture of both lights. Alternatively, pixel-polarized image sensors can be used with orthogonally polarized infrared and visible lights. This means some of the pixels only see infrared and others only see visible light, depending on the polarization. Alternatively, image sensors with pixel-level infrared filters can be used, so that some of the pixels can sense infrared light while others cannot or are less sensitive to it. In a 3-chip design, the 3 image sensors can be mechanically or optically offset such that their respective infrared pixels fill the void for each other and form a less sub-sampled infrared image. Such a design is shown in Fig. 3e. Fig. 3e shows a schematic diagram of filters being applied to three sensors for simultaneously sensing visible light and infrared light. For example, there may be one infrared pixel in every 2-by-2 square of pixels (marked by X in Fig. 3e). In each sensor, the infrared pixel may be in another position of the 2-by-2 square (see Fig. 3e 360-364), so in the resulting 2-by-2 square 366, three of the four pixels have been used to record infrared light.

Alternatively, light sources can be controlled to synchronize with the image sensors so that in the sequence of images captured, every other image is illuminated by infrared, and the rest by visible light.

In the following, various aspects of the image processing (to generate the processed image data) to utilize the infrared component is discussed. For example, where the infrared light component is sensed separately from visible light, the color reproduction from visible light image(s) might not be affected by the introduction of infrared illumination. Each infrared image can be used either as a "Detail" image or das a "Guide" image. When being used as a "Detail" image, the infrared image may be high-pass filtered to retain only the high spatial frequency content (HF), while the visible light image is low-pass filtered to retain only the low spatial frequency content (LF). The HF image may be added to the LF image to form the final output image (i.e. the processed image data). Because the infrared image is allowed to have higher light level and thus higher signal-to-noise ratio, the output image can have reduced high frequency noise. When being used as a "Guide" image, each pixel in the infrared image may be compared against its neighboring pixels to obtain a weighting matrix. More similar neighbors may have higher weights. These weighting matrices may be used as spatially varying filter kernels to filter the visible light image (in the 1-chip case, either before or after demosaicing) to reduce noise. Because the infrared image is less noisy than the visible one, it serves as a reliable guide for the similarity-based filtering of the visible light image to reduce noise.

In some cases, as mentioned above, the infrared light component is sensed using the same sensor(s) as visible light. In general, if infrared and visible light are captured simultaneously and are inseparable at sensor output, a nonlinear color correction process can be used to reproduce natural colors, in contrast to the approach chosen for the proposed concept. Images of sample tissues and color swatches may be captured in pairs, one with infrared and one without infrared illumination, at the same visible light level. Color correspondence can then be established for the nonlinear color correction process. This nonlinear process can take forms such as a three-dimensional lookup table or a hue-based color wheel. Alternatively, a weighted average of the three color components (each with infrared mixed within) can be computed. This weighted average is considered the luminance component. Color differences are then computed, filtered, and color-corrected using matrix multiplication (linear method) or a nonlinear process like the one described above. The luminance and color differences may be combined and linearly converted to red-green-blue.

In various examples of the present disclosure, if pixel-polarized image sensors are used, the differently polarized pixel subsets may be processed separately as the infrared and visible light sub-images. The infrared image can then be used either as the "detail" image or "guide" image as described above to generate the processed image data. Similarly, if the image sensors include pixel-level infrared filtration, in a 3-chip design, the infrared images can be interpolated separately from the visible light ones. In a 1-chip design, the demosaicing algorithm can treat the infrared pixels as a fourth color and perform four-color interpolation to obtain color + infrared images. The infrared image can then be used either as the "detail" image or "guide" image as described above to generate the processed image data. If infrared and visible light are captured separately in temporally alternating images, previous images can be compared with the current to estimate motion in the scene and register the pixels of the previous images to the current image (i.e., dense optical flow). The infrared image can then be used either as the "detail" image or "guide" image as described above to generate the processed image data.

More details and aspects of the more detailed examples are mentioned in connection with the proposed concept or one or more examples described above or below (e.g. Fig. 1a to 2). The proposed concept may comprise one or more additional optional features corresponding to one or more aspects of the proposed concept or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a microscope comprising a system as described in connection with one or more of the Figs. 1a to 3e. Alternatively, a microscope may be part of or connected to a system as described in connection with one or more of the Figs. 1 to 3e. Fig. 4 shows a schematic illustration of a system 400 configured to perform a method described herein. The system 400 comprises a microscope 410 and a computer system 420. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of reference Signs

- 100: Microscope system
- 105: Base unit / stand
- 110: System
- 112: Interface
- 114: One or more processors
- 116: One or more storage devices
- 120: Microscope
- 122: Ocular displays
- 130: Illumination system
- 140: Heads-up display
- 150: Arm
- 210: Emitting light
- 220: Obtaining image data
- 230: Processing the image data
- 240: Generating processed image data
- 250: Generating a display signal
- 310: Visible light
- 320: Infrared light
- 330: Separate optical imaging sensor for sensing infrared light
- 340: Optical imaging sensors in three-chip configuration
- 350: Optical imaging sensor in one-chip configuration
- 360, 362, 364: Position of pixels for sensing infrared light
- 366: Position of pixels for sensing infrared light in combined image
- 400: System
- 410: Microscope
- 420: Computer system

## Claims

1. A microscope system (100) for use in eye surgery, the microscope system comprising:
a microscope (120) comprising at least one optical imaging sensor (330; 340; 350);
an illumination system (130) for illuminating the eye, wherein the illumination system is configured to emit light in a near infrared wavelength band and in a visible light wavelength band; and
one or more processors (114) configured to:
obtain image data of the least one optical imaging sensor of the microscope, the image data representing infrared light and visible light sensed by the at least one optical imaging sensor,
process the image data to determine a first component representing visible light and a second component representing infrared light,
generate processed image data based on the first component and based on the second component of the image data, and
generate a display signal for a display device (122; 140) based on the processed image data.

2. The microscope system according to claim 1, wherein the illumination system is configured to provide the illumination through a co-axial light path of the microscope,
or wherein the illumination system is configured to provide the illumination via an endo-illuminator.

3. The microscope system according to one of the claims 1 to 4, wherein the image data comprises the first component representing visible light and the second component representing infrared light separately from each other.

4. The microscope system according to one of the claims 1 or 2, wherein the one or more processors are configured to separate the first and the second component within the image data.

5. The microscope system according to one of the claims 1 to 4, wherein the image data represents infrared light and visible light reflected by the eye and subsequently sensed by the at least one optical imaging sensor.

6. The microscope system according to one of the claims 1 to 5, wherein the one or more processors are configured to spatially low-pass filter the first component and to spatially high-pass filter the second component, and to generate the processed image data by combining the spatially low-pass filtered first component and the spatially high-pass filtered second component.

7. The microscope system according to one of the claims 1 to 6, wherein the one or more processors are configured to determine a weighting matrix based on the second component, and to use the weighting matrix as spatially varying filter kernels for filtering the first component, and to generate the processed image data based on the filtered first component.

8. The microscope system according to one of the claims 1 to 7, wherein the microscope comprises at least one optical imaging sensor (340; 350) for sensing visible light and a separate optical imaging sensor (330) for sensing infrared light.

9. The microscope system according to one of the claims 1 to 7, wherein the microscope comprises at least one optical imaging sensor (340; 350) for simultaneously sensing visible light and infrared light.

10. The microscope system according to claim 9, wherein the at least one optical imaging sensor comprises a polarization filter configured to admit light having a first polarization to a first subset of pixels of the at least one optical imaging sensor and to admit light having a second polarization to a second subset of pixels of the at least one optical imaging sensor, the first polarization being orthogonal to the second polarization, wherein the illumination system is configured to emit the light in the visible light wavelength band having the first polarization, and to emit the light in the near infrared wavelength band having the second polarization.

11. The microscope system according to claim 9, wherein the at least one optical imaging sensor comprises an infrared light filter configured to admit infrared light to a subset of pixels of the at least one optical imaging sensor.

12. The microscope system according to claim 11, comprising three optical imaging sensors, wherein the infrared light filter is configured to admit infrared light to a subset of pixels of each of the three optical imaging sensors, such that infrared light is admitted to a different subset of pixels of each of the three optical imaging sensors.

13. A system (110) for a microscope system (100) for use in eye surgery, the system comprising one or more processors and one or more storage devices, wherein the system is configured to:
obtain image data of at least one optical imaging sensor of a microscope of the microscope system, the image data representing infrared light and visible light sensed by the at least one optical imaging sensor;
process the image data to determine a first component representing visible light and a second component representing infrared light;
generate processed image data based on the first component and based on the second component of the image data; and
generate a display signal for a display device based on the processed image data.

14. A method for a system for a microscope system, the method comprising:
obtaining (220) image data of at least one optical imaging sensor of the microscope, the image data representing infrared light and visible light sensed by the at least one optical imaging sensor of a microscope of the microscope system;
processing (230) the image data to determine a first component representing visible light and a second component representing infrared light;
generating (240) processed image data based on the first component and based on the second component of the image data; and
generating (250) a display signal based on the processed image data.

15. A computer program with a program code for performing the method according to claim 14 when the computer program is executed on a processor.
